# EUROPEAN PATENT SPECIFICATION

(11) **EP 2 732 044 B1**
(45) Date of publication and mention of the grant of the patent: **07.09.2016**
(21) Application number: 12810673.9
(22) Date of filing: 12.07.2012
(51) Int. Cl.: A61K 38/08, A61K 38/10, A61P 35/00

(54) **NOVEL ZINC FINGER-LIKE PEPTIDE COMPOSITIONS AS POTENT AGENTS IN CANCER PREVENTION AND TREATMENT**
NEUE ZINKFINGERARTIGE PEPTIDZUSAMMENSETZUNGEN ALS STARKE WIRKSTOFFE FÜR DIE VORBEUGUNG UND BEHANDLUNG VON KARZINOMEN
NOUVELLES COMPOSITIONS DE PEPTIDES EN DOIGTS DE ZINC UTILISÉES COMME AGENTS PUISSANTS POUR LA PRÉVENTION ET LE TRAITEMENT DU CANCER

(30) Priority: 12.07.2011 TW 100124615
(43) Date of publication of application: 21.05.2014
(73) Proprietor: National Cheng Kung University, Tainan City 701 (TW)
(72) Inventor: CHANG, Nan-Shan, Owega, New York 13827 (US); LEE, Ming-Hui, Tainan City 701 (TW); CHANG, Jean-Yun, Tainan City 701 (TW); LIN, Sing-Ru, Tainan City 701 (TW); SU, Wan-Pei, Tainan City 701 (TW)
(74) Representative: Wilkins, Christopher George
(86) International application number: PCT/US2012/046390
(87) International publication number: WO 2013/009948

(56) References cited:
- WO-A2-02/46412
- JP-A- 2004 315 423
- S PLANEL ET AL: "A novel concept in antiangiogenic and antitumoral therapy: multitarget destabilization of short-lived mRNAs by the zinc finger protein ZFP36L1", ONCOGENE, vol. 29, no. 45, 11 November 2010 (2010-11-11), pages 5989-6003, XP055015865, ISSN: 0950-9232, DOI: 10.1038/onc.2010.341
- HAN YOUQI ET AL: "The zinc finger domain of Wilms' tumor 1 suppressor gene (WT1) behaves as a dominant negative, leading to abrogation of WT1 oncogenic potential in breast cancer cells", BREAST CANCER RESEARCH, CURRENT SCIENCE, LONDON, GB, vol. 9, no. 4, 16 July 2007 (2007-07-16), page R43, XP021031132, ISSN: 1465-5411, DOI: 10.1186/BCR1743
- CHANG G T G ET AL: "Characterization of a Zinc-Finger Protein and Its Association with Apoptosis in Prostate Cancer Cells", JOURNAL OF THE NATIONAL CANCER INSTITUTE, OXFORD UNIVERSITY PRESS, GB, vol. 92, no. 17, 6 September 2000 (2000-09-06), pages 1414-1421, XP003009569, ISSN: 0027-8874, DOI: 10.1093/JNCI/92.17.1414
- NAN-SHAN CHANG: 'Naturally occurring peptide Zfra inactivates tumor suppressors by covalent binding: An act of Zfration' DTIC, [Online] September 2009, Retrieved from the Internet: <URL:www.dtic.mil/dtic/tr/fulltext/u2/a5126 51.pdf> [retrieved on 2015-07-30]

### SEQUENCE LISTING

<110> National Cheng Kung University
<120> NOVEL ZINC FINGER-LIKE PEPTIDE COMPOSITIONS AS POTENT AGENTS IN CANCER PREVENTION AND TREATMENT
<130> 20.118964
<140> EP12810673.9
   <141> 2012-07-12
<150> TW 100124615
   <151> 2011-07-12
<160> 9
<170> PatentIn version 3.5
<210> 1
   <211> 7
   <212> PRT
   <213> Artificial sequence
<220>
   <223> synthesized peptide
<400> 1
   Arg Arg Ser Ser Ser Cys Lys
   1               5
<210> 2
   <211> 31
   <212> PRT
   <213> Artificial sequence
<220>
   <223> synthesized peptide
<400> 2
   Met Ser Ser Arg Arg Ser Ser Ser Cys Lys Tyr Cys Glu Gln Asp Phe
   1               5                   10                  15
   Arg Ala His Thr Gln Lys Asn Ala Ala Thr Pro Phe Leu Ala Asn
               20                  25                  30
<210> 3
   <211> 21
   <212> DNA
   <213> Artificial sequence
<220>
   <223> synthesized DNA
<400> 3
   agaaggtcgt cttcttgtaa a 21
<210> 4
   <211> 93
   <212> DNA
   <213> Artificial sequence
<220>
   <223> synthesized DNA
<400> 4
   atgagcagca gaaggtcgtc ttcttgtaaa tattgtgaac aggacttccg agcacacaca        60
   cagaagaatg cggccacacc cttcctagcc aac                                     93
<210> 5
   <211> 15
   <212> PRT
   <213> Artificial sequence
<220>
   <223> synthesized peptide
<400> 5
   Met Ser Ser Arg Arg Ser Ser Gly Cys Lys Tyr Cys Glu Gln Asp
   1               5                   10                  15
<210> 6
   <211> 6
   <212> PRT
   <213> Artificial sequence
<220>
   <223> synthesized peptide
<400> 6
   Arg Ser Ser Ser Cys Lys
   1 5
<210> 7
   <211> 93
   <212> DNA
   <213> Artificial sequence
<220>
   <223> synthesized DNA
<400> 7
   atgagcagca gaaggtcgtc tggctgtaaa tattgtgaac aggacttccg agcacacaca 60
   cagaagaatg cggccacacc cttcctagcc aac 93
<210> 8
   <211> 15
   <212> PRT
   <213> Artificial sequence
<220>
   <223> synthesized peptide
<400> 8
   Met Ser Ser Arg Arg Ser Ser Ser Cys Lys Tyr Cys Glu Gln Asp
   1 5 10 15
<210> 9
   <211> 16
   <212> PRT
   <213> Artificial sequence
<220>
   <223> synthesized peptide
<400> 9
   Phe Arg Ala His Thr Gln Lys Asn Ala Ala Thr Pro Phe Leu Ala Asn
   1 5 10 15

### SEQUENCE LISTING

<110> National Cheng Kung University
<120> NOVEL ZINC FINGER-LIKE PEPTIDE COMPOSITIONS AS POTENT AGENTS IN CANCER PREVENTION AND TREATMENT
<130> 20.118964
<140> EP12810673.9
   <141> 2012-07-12
<150> TW 100124615
   <151> 2011-07-12
<160> 9
<170> PatentIn version 3.5
<210> 1
   <211> 7
   <212> PRT
   <213> Artificial sequence
<220>
   <223> synthesized peptide
<400> 1
   Arg Arg Ser Ser Ser Cys Lys
   1               5
<210> 2
   <211> 31
   <212> PRT
   <213> Artificial sequence
<220>
   <223> synthesized peptide
<400> 2
   Met Ser Ser Arg Arg Ser Ser Ser Cys Lys Tyr Cys Glu Gln Asp Phe
   1               5                   10                  15
   Arg Ala His Thr Gln Lys Asn Ala Ala Thr Pro Phe Leu Ala Asn
               20                  25                  30
<210> 3
   <211> 21
   <212> DNA
   <213> Artificial sequence
<220>
   <223> synthesized DNA
<400> 3
   agaaggtcgt cttcttgtaa a 21
<210> 4
   <211> 93
   <212> DNA
   <213> Artificial sequence
<220>
   <223> synthesized DNA
<400> 4
   atgagcagca gaaggtcgtc ttcttgtaaa tattgtgaac aggacttccg agcacacaca 60
   cagaagaatg cggccacacc cttcctagcc aac 93
<210> 5
   <211> 15
   <212> PRT
   <213> Artificial sequence
<220>
   <223> synthesized peptide
<400> 5
   Met Ser Ser Arg Arg Ser Ser Gly Cys Lys Tyr Cys Glu Gln Asp
   1 5 10 15
<210> 6
   <211> 6
   <212> PRT
   <213> Artificial sequence
<220>
   <223> synthesized peptide
<400> 6
   Arg Ser Ser Ser Cys Lys
   1 5
<210> 7
   <211> 93
   <212> DNA
   <213> Artificial sequence
<220>
   <223> synthesized DNA
<400> 7
   atgagcagca gaaggtcgtc tggctgtaaa tattgtgaac aggacttccg agcacacaca 60
   cagaagaatg cggccacacc cttcctagcc aac 93
<210> 8
   <211> 15
   <212> PRT
   <213> Artificial sequence
<220>
   <223> synthesized peptide
<400> 8
   Met Ser Ser Arg Arg Ser Ser Ser Cys Lys Tyr Cys Glu Gln Asp
   1               5                   10                  15
<210> 9
   <211> 16
   <212> PRT
   <213> Artificial sequence
<220>
   <223> synthesized peptide
<400> 9
   Phe Arg Ala His Thr Gln Lys Asn Ala Ala Thr Pro Phe Leu Ala Asn
   1               5                   10                  15

## Description

### BACKGROUND OF THE INVENTION

### 1. Field of the Invention

The present invention relates to a zinc finger-like peptide for treating cancer, a pharmaceutical composition containing the zinc finger-like peptide and a method for treating cancer.

### 2. Description of Related Art

Foods or food additives, and environmental pollutions have been a source of contention as a cause or catalyst for promoting cancer in recent years. Not coincidentally, the same event is happening as well in the developed countries and around the world, positing as an alarming sign that the incidence rates of cancers are quite high. According to the data published by the American Cancer Society, cancer is being proved to be the most significant threat to public health.

The general methods for treating cancer include surgery, radiotherapy, chemotherapy and immune therapy. In recent years, the development of several therapeutic agents has lead to cancer treatments through new anti-cancer mechanisms, and it has been proven that the survival rate of patients can be increased by treating them with these therapeutic agents. Generally, the therapeutic agents can treat cancers through inhibition of cell cycle progression, angiogenesis, farnesyl transferase, and tyrosine kinases.

Although it is known that certain agents exhibit therapeutic effects on cancer, these agents do have their limitations. For example, "Gefitinib" is a drug for inhibiting non-small cell lung cancer, but it fails to cure in most cases. Also, it has no effects on blocking the progression of breast and colorectal cancers. In addition, the therapeutic effects of the anti-cancer drugs also depend on the locations of tumor cells, genetic variations of patients, and the side effects of drugs. Furthermore, cancer cells may become malignant and spread from its original sites to target organs via the lymphatic system or bloodstream, thereby establishing metastatic cancers.

Since the risk of developing cancer generally increases with age, the occurrence rates of cancer go up as more people live to an old age and as mass lifestyle changes. Hence, there is a long unfulfilled need to provide new agents for cancer treatment and prevention.

### SUMMARY OF THE INVENTION

The present invention is defined by the claims.

The object of the present invention is to provide a zinc finger-like peptide and a pharmaceutical composition containing the zinc finger-like peptide for use in treating cancer if administered intravenously.

Another object of the present disclosure is to provide a zinc finger-like peptide expression plasmid, a pharmaceutical composition containing the zinc finger-like peptide and a method for treating cancer, wherein the zinc finger-like peptide expression plasmid can express a zinc finger-like peptide for treating cancer.

To achieve the object, the zinc finger-like peptide for treating cancer comprises: at least seven amino acids, wherein the sequence of the at least seven amino acids is represented by SEQ ID NO: 1.

In addition, the zinc finger-like peptide expression plasmid for treating cancer comprises: a DNA sequence for expressing the aforementioned zinc finger-like peptide.

To achieve the object, the zinc finger-like peptide for treating cancer comprises: at least seven amino acids, wherein the sequence of the at least seven amino acids is represented by SEQ ID NO: 1 (RRSSSCK).

In addition, the zinc finger-like peptide expression plasmid for treating cancer comprises: a DNA sequence for expressing the aforementioned zinc finger-like peptide, which has 100% identity to a sequence represented by SEQ ID NO: 3 (agaaggtcgt cttcttgtaa a).

The present invention provides a zinc finger-like peptide, which is a small molecule peptide similar to a zinc finger motif. The amino-acid length of the peptide is not particularly limited, and the peptide can be constituted of 4-200 amino acids. Preferably, the peptide contains 4-100 amino acids. More preferably, the peptide contains 5-70 amino acids. Most preferably, the peptide contains 6-45 amino acids. Particularly preferably, the peptide contains 7-15 amino acids. The zinc finger-like peptide for use in treating cancer comprises at least seven amino acids, wherein the sequence of the at least seven amino acids is represented by SEQ ID NO: 1. Most preferably, the sequence of the zinc finger-like peptide is SEQ ID NO: 1.

The zinc finger-like peptide of the present invention may be represented by SEQ ID NO: 2 (MSSRRSSSCKYCEQDFRAHTQKNAATPFLAN).

The DNA sequences shown in SEQ ID NOs: 3 and 4 are respectively coded into the amino acid sequences shown in SEQ ID NOs: 1 and 2. The DNA sequences shown in SEQ ID NO 4 is listed as the following: atgagcagca gaaggtcgtc ttcttgtaaa tattgtgaac aggacttccg agcacacaca cagaagaatg cggccacacc cttcctagcc aac. In the present invention, cDNA having DNA sequences shown in SEQ ID NO: 3 or 4 can be inserted into a vector to obtain the zinc finger-like peptide expression plasmid, which can express peptide having amino acid sequence shown in SEQ ID NO: 1 or 2 *in vitro* or *in vivo.*

The sequence of the aforementioned zinc finger-like peptide can be derived from any zinc finger-like peptide of different species. Preferably, the zinc finger-like peptide of the present invention is derived from human or mouse. In addition, the zinc finger-like peptide may be obtained through peptide synthesis, or expression of cDNA of zinc finger-like peptide from human or mouse. In one aspect of the present invention, the zinc finger-like peptide of the present invention is obtained through peptide synthesis.

The expression plasmid of the present disclosure can be any plasmid capable of expressing the zinc finger-like peptide of the present invention. Preferably, the expression plasmid used in the present invention is pCR3.1 (available from Invitrogen) or pEGFPC1 (available from Clontech). The host for the zinc finger-like peptide of the present invention is not particularly limited, as long as it can express the cDNA inserted into the vector. Examples of the host may include: bacteria, mammalian cells or tumor cells. Preferably, the zinc finger-like peptide expression plasmid of the present invention is transfected into mammalian cells or tumor cells to express the zinc finger-like peptide.

In the zinc finger-like peptide containing at least seven amino acids of the present invention, the sequence thereof may contain at least two duplicate amino acid residues with similar chemical properties. For example, the sequence thereof may contain at least two duplicate hydrophilic amino acid residues such as aspartic acid, glutamine, serine, threonine, or cysteine; at least two duplicate amino acid residues with alcoholic groups, such as threonine, serine or tyrosine; or at least two duplicate amino acid residues with thio-groups such as cysteine. In the present invention, the zinc finger-like peptide at least contains two cysteine and one histidine to form a zinc finger-like peptide with C2H2 motif.

In addition, the zinc finger-like peptide expression plasmid of the present invention may further comprise a vector connecting to the DNA sequence for expressing the zinc finger-like peptide of the present invention. The type of the vector is not particularly limited by number, and can be any vector generally used in the art. For example, the vector may be derived from pEGFP or pECFP, which is generally used to transfect into mammalian cells such as human cell lines for expressing inserted cDNAs.

Furthermore, the zinc finger-like peptide or the expression plasmid of the present invention may be encapsulated into liposome. Preferably, the expression plasmid is co-transfected into hosts with liposome to express the zinc finger-like peptide.

The zinc finger-like peptide of the present invention has similar structure to zinc-finger motif. In addition, the peptide of the present invention may selectively contain several duplicate amino acid residues with similar chemical properties or several duplicate identical amino acid residues to form a peptide motif with specific bondings. Furthermore, the zinc finger-like peptide can self-polymerize without adding any catalytic agents. The zinc finger-like peptide of the present invention also can bind to proteins related to apoptosis. Hence, it can inhibit the growth of tumor cells, and can be used to treat or prevent cancers.

The present invention further provides a pharmaceutical composition for treating cancer, which comprises: an effective amount of the aforementioned zinc finger-like peptide or zinc finger-like peptide expression plasmid; and a pharmaceutically acceptable carrier.

In addition, the present invention also provides a method for treating cancer, which comprises: administering the aforementioned zinc finger-like peptide, the aforementioned zinc finger-like peptide expression plasmid, or the aforementioned pharmaceutical composition to a subject in need thereof.

The zinc finger-like peptide of the present invention may be applied to various cancers, such as bladder cancer, bone cancer, brain cancer, breast cancer, cervical caner, colon cancer, endometrial cancer, esophageal cancer, leukemia, liver cancer, lymphoma, kidney cancer, osteosarcoma, ovarian cancer, pancreatic cancer, prostate cancer, skin cancer including basal and squamous cell carcinoma and melanoma, small intestine cancer, stomach cancer, thymus cancer and thyroid cancer, but the scope of applicability of the present invention is not limited thereto. Preferably, the zinc finger-like peptide of the present invention is used to treat breast cancer, colon cancer, esophageal cancer, leukemia, lymphoma, liver cancer, lung cancer, ovarian cancer, pancreatic cancer, prostate cancer, skin cancer, stomach cancer or thyroid cancer. More preferably, the zinc finger-like peptide of the present invention is used to treat breast cancer, colon cancer, lung cancer, lymphoma, prostate cancer or skin cancer. Most preferably, the zinc finger-like peptide of the present invention is used to treat brain cancer, breast cancer, prostate cancer or skin cancer.

The zinc finger-like peptide of the present invention can inhibit proliferation, growth, invasion and metastasis of tumor cells.

The zinc fmger-like peptide and the pharmaceutical composition for treating cancer of the present invention is to be administered via parenteral, Namely injection.

According to the pharmaceutical composition of the present invention, the term "pharmaceutically acceptable carrier" means that the carrier must be compatible with the active ingredients (and preferably, capable of stabilizing the active ingredients) and not be deleterious to the subject to be treated. The carrier may be at least one selected from the group consisting of active agents, adjuvants, dispersants, wetting agents and suspending agents. The example of the carrier may be microcrystalline cellulose, mannitol, glucose, non-fat milk powder, polyethylene, polyvinylprrolidone, starch or a combination thereof.

In addition, the term "treating" used in the present invention refers to the application or administration of the zinc finger-like peptide or the pharmaceutical composition containing the zinc finger-like peptide to a subject with symptoms or tendencies of suffering from cancer in order to cure, heal, alleviate, relieve, alter, remedy, ameliorate, improve, prevent or affect the symptoms or tendencies of cancers. Furthermore, "an effective amount" used herein refers to the amount of each active ingredients such as the zinc finger-like peptide required to confer therapeutic effect on the subject. The effective amount may vary according to the route of administration, excipient usage, and co-usage with other active ingredients.

Other objects, advantages, and novel features of the invention will become more apparent from the following detailed description when taken in conjunction with the accompanying drawings.

### BRIEF DESCRIPTION OF THE DRAWINGS

FIG. 1A is a curve representing the tumor volumes of mice in the experimental group according to Example 1 of the present invention;
FIG. 1B is a curve representing the tumor volumes of mice in the control group according to Example 1 of the present invention;
FIG. 2 shows curves representing the tumor volumes of mice according to Example 2 of the present invention;
FIG. 3A is a curve representing the tumor volumes of mice in the experimental group according to Example 3 of the present invention;
FIG 3B is a curve representing the tumor volumes of mice in the control group according to Example 3 of the present invention;
FIG. 4 shows curves representing the tumor volumes of mice according to Example 4 of the present invention;
FIG. 5 shows curves representing the tumor volumes of mice according to Example 5 of the present invention;
FIG. 6A is a curve representing the tumor volumes of mice in the experimental group according to Example 6 of the present invention;
FIG. 6B is a curve representing the tumor volumes of mice in the control group according to Example 6 of the present invention;
FIG. 7A is a curve representing the tumor volumes of mice in the experimental group according to Example 7 of the present invention;
FIG. 7B is a curve representing the tumor volumes of mice in the control group according to Example 7 of the present invention;
FIG. 8 shows curves representing the tumor volumes of mice according to Example 8 of the present invention;
FIG. 9A is a curve representing the tumor volumes of mice in the experimental group according to Example 9 of the present invention;
FIG. 9B is a curve representing the tumor volumes of mice in the control group according to Example 9 of the present invention;
FIG. 10A is a curve representing the tumor volumes of mice in the experimental group according to Example 10 of the present invention;
FIG. 10B is a curve representing the tumor volumes of mice in the control group according to Example 10 of the present invention;
FIG. 11A is a curve representing the tumor volumes of mice in the control group according to Example 11 of the present invention;
FIG. 11B is a curve representing the tumor volumes of mice in the experimental group 1 according to Example 11 of the present invention; and
FIG. 11C is a curve representing the tumor volumes of mice in the experimental group 1 according to Example 11 of the present invention.

The DNA sequence and the amino acid sequence of the zinc finger-like peptide used in the following examples are listed in the following table 1.

**Table 1.**

| SEQ ID NO: | DNA/amino acid sequence |
|---|---|
| SEQ ID NO: 1 | RRSSSCK |
| SEQ ID NO: 2 | MSSRRSSSCKYCEQDFRAHTQKNAATPFLAN |
| SEQ ID NO: 3 | agaaggtcgt cttcttgtaa a |
| SEQ ID NO: 4 | |
| SEQ ID NO: 5 | MSSRRSS*G*CKYCEQD (serine mutation) |
| SEQ ID NO: 6 | RSSSCK (cysteine deletion) |
| SEQ ID NO: 7 | atgagcagca gaaggtcgtc t*ggc*tgtaaa tattgtgaac aggacttccg |
| | agcacacaca cagaagaatg cggccacacc cttcctagcc aac |
| SEQ ID NO: 8 | MSSRRSSSCKYCEQD |
| SEQ ID NO: 9 | FRAHTQKNAATPFLAN |

In the following examples and comparative examples, 8-week nude mice were used as host subjects to perform *in vivo* experiments. The nude mice were placed in room temperature and suitable humidity, and supplied with sterile water and standard food for 2 consecutive weeks before performing experiments. Then, the nude mice were injected with zinc finger-like peptide and tumor cells. The detailed processes for the experiments are described as follows.

First, manually synthesized zinc finger-like peptide (Genemed Synthesis Inc., San Antonio, TX, USA) was resuspended in degassed purified water to obtain 1-5 mM of zinc finger-like peptide solution (100 µl). Herein, the solution for suspending the zinc finger-like peptide is not particularly limited. Preferably, the zinc finger-like peptide is resuspended in degassed purified water, in order to prevent the self-polymerization of the zinc finger-like peptide and loss its ability to inhibit tumor cell growth.

### [Example 1]

### Experimental group

Herein, zinc finger-like peptide with sequence shown in SEQ ID NO: 2 was injected into nude mice.

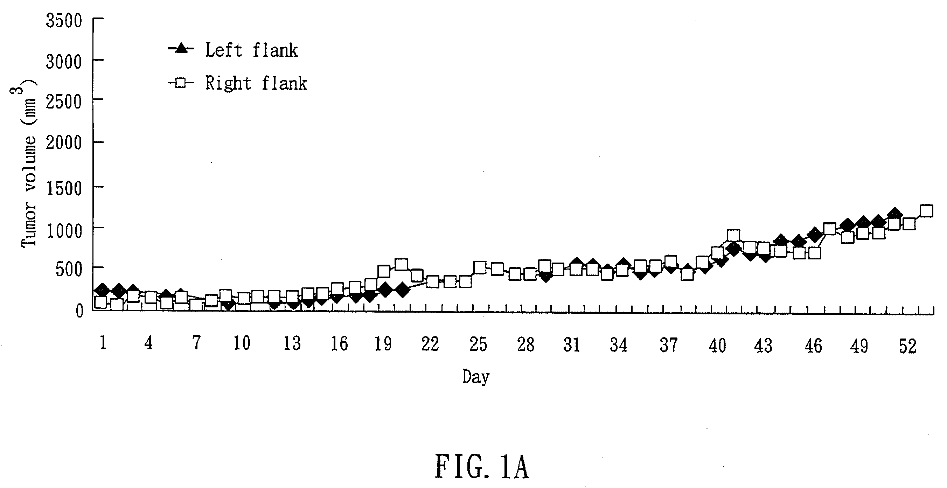

In particularly, nude mice were injected with an intravenous injection of peptide (2 mM, 100 µl phosphate-buffered saline or PBS) via tail veins per week for 3 consecutive weeks, followed by inoculating basal cell carcinoma (BCC) cells (2 million cells / 100 µl PBS) at both the right and left flanks. The tumor volumes (mm³) were observed daily during the whole experiment. The result is shown in FIG. 1A, which is a curve representing the tumor volumes of mice.

### Control group

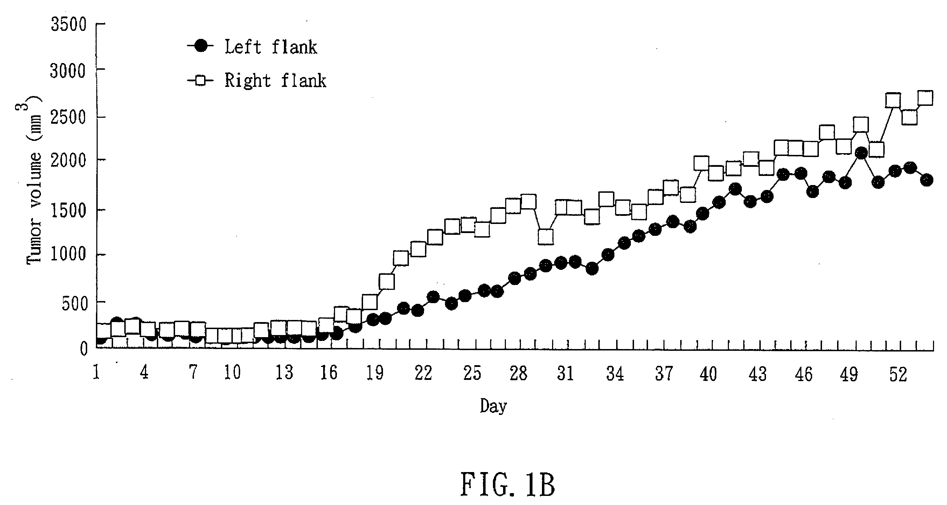

The process of the present control group was the same as that of the experimental group, except that the zinc finger-like peptide of the experimental group was substituted with PBS buffer (100 µl) in the present control group. The result is shown in FIG. 1B, which is a curve representing the tumor volumes of mice.

As shown in FIG. 1A and FIG. 1B, the zinc finger-like peptide inhibited the growth of skin cancer cell on nude mice when the mice were pre-injected with zinc finger-like peptide (experimental group). However, the tumor volumes of mice without injection of zinc finger-like peptide (control group) were much larger than those of the experimental group. These results indicate that the zinc finger-like peptide is effective on treating and preventing skin basal cell carcinoma.

### [Example 2]

### Experimental group 1

In the present experimental group, zinc finger-like peptide with sequence shown in SEQ ID NO: 1 was injected into nude mice.

Nude mice were injected with an intravenous injection of peptide (2 mM, 100 µl PBS) via tail veins per week for 3 consecutive weeks, followed by inoculating BCC cells (2 million cells / 100 µl PBS) at both the right and left flanks. 2 months later, boost injection of peptide was carried out per week for 3 consecutive weeks. The tumor volumes (mm³) were daily observed during the whole experiment.

### Experimental group 2

The process of the present experimental group was the same as that of the experimental group 1, except that the zinc finger-like peptide with sequence shown in SEQ ID NO: 1 of the experimental group 1 was substituted with zinc finger-like peptide with sequence shown in SEQ ID NO: 5 in the present experimental group.

### Control group

The process of the present control group was the same as that of the experimental group 1, except that the zinc finger-like peptide of the experimental group 1 was substituted with H₂O (100 µl) in the present control group.

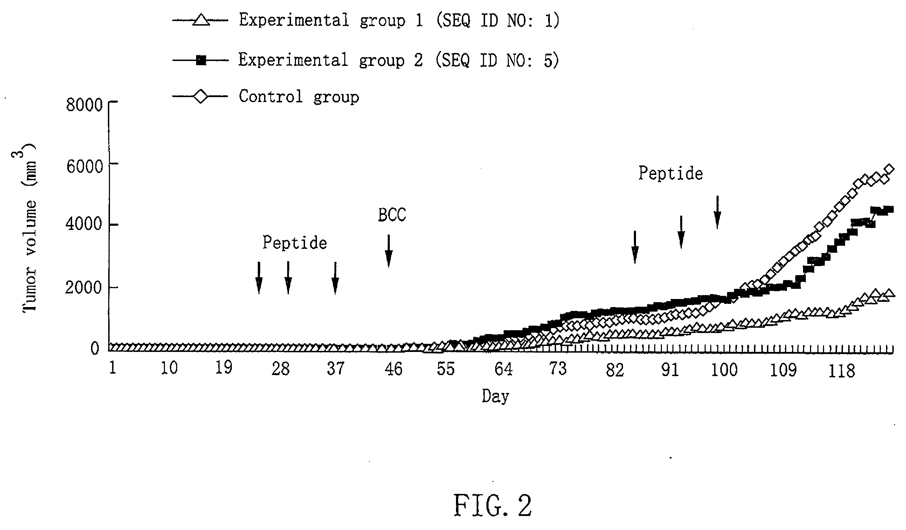

The results of the present example are shown in FIG. 2, which shows curves representing the tumor volumes of mice of the experimental group 1, the experimental group 2 and the control group of the present example. As shown in FIG. 2, the zinc finger-like peptide with sequence shown in SEQ ID NO: 1 (experimental group 1) can prevent and inhibit the growth of cancer cell, and has effect on skin cancer treatment. However, when the fifth amino acid, serine of SEQ ID NO: 1 is replaced with glycine (i.e. the zinc finger-like peptide with sequence shown in SEQ ID NO: 5), the therapeutic effect of the zinc finger-like peptide is decreased. However, the zinc finger-like peptide used in the experimental group 2 still has better treatment effect than H₂O used in the control group.

### [Example 3]

### Experimental group

Herein, zinc finger-like peptide with sequence shown in SEQ ID NO: 1 is injected into nude mice.

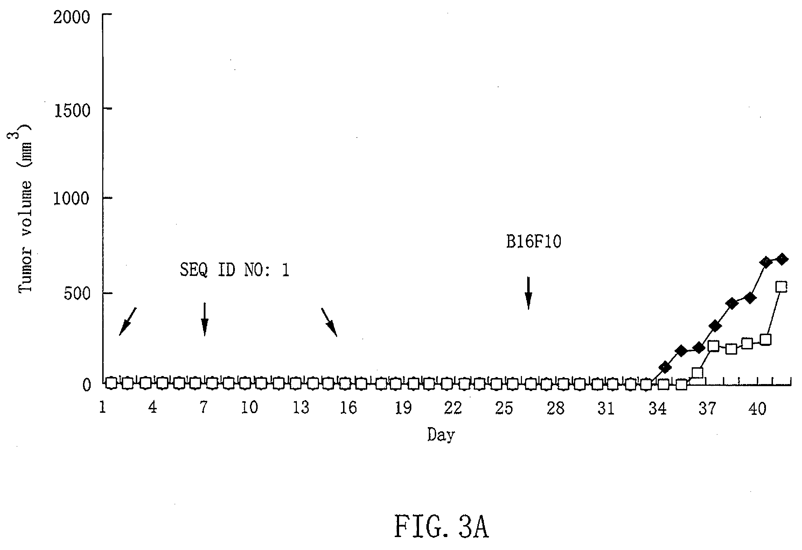

With this experimental group, nude mice received an intravenous injection of peptide (2 mM, 100 µl sterile water) via tail veins per week for 3 consecutive weeks, followed by inoculation of malignant melanoma B16F10 (2 million cells / 100 µl PBS) at both the right and left flanks. The tumor volumes (mm³) were observed daily during the whole experiment. Shown in FIG. 3A is the result representing the tumor volumes of mice of the present experimental group.

### Control group

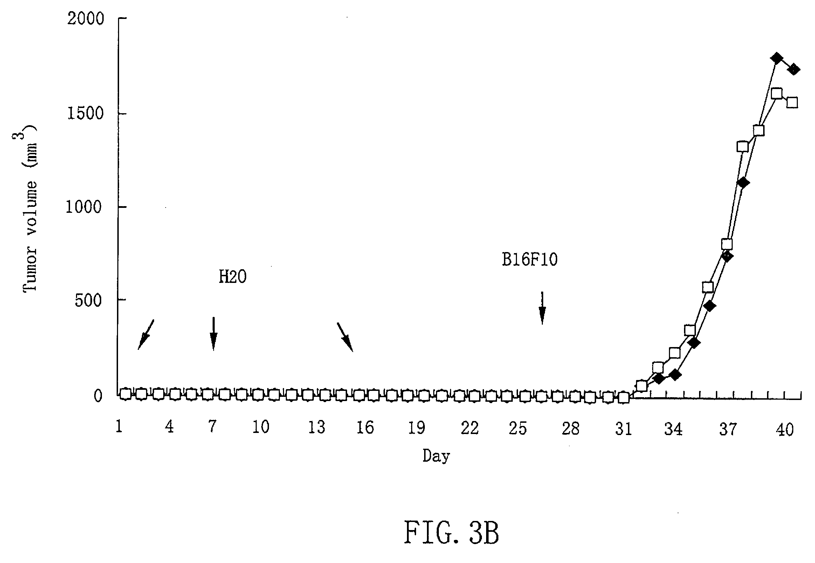

The process of the present control group was the same as that of the experimental group, except that the zinc finger-like peptide of the experimental group was substituted with H₂O (100 µl) in the present control group. The result is shown in FIG 3B, which is a curve representing the tumor volumes of mice of the present control group.

As shown in FIG. 3A and FIG. 3B, the zinc finger-like peptide can inhibit the growth of skin melanoma on nude mice when the mice were pre-injected with zinc finger-like peptide (experimental group). However, the tumor volumes of mice without injection of zinc finger-like peptide (control group) were much larger than those of the experimental group. These results indicate that the zinc finger-like peptide exhibit dramatic effects on treating and preventing malignant melanoma.

### [Example 4]

Nude mice received an intravenous injection of peptide (2 mM, 100 µl) via tail veins per week for 2 consecutive weeks, followed by inoculating malignant melanoma B16F10 (2 million cells / 100 µl PBS) at both the right and left flanks. The tumor volumes (mm³) were observed daily during the whole experiment.

### Experimental group 1

In the present experimental group, zinc finger-like peptide with sequence shown in SEQ ID NO: 1 was injected into nude mice.

### Experimental group 2

In the present experimental group, zinc finger-like peptide with sequence shown in SEQ ID NO: 1, in which the fifth amino acid, serine was phosphorylated, and injected into nude mice.

### Experimental group 3

In the present experimental group, zinc finger-like peptide with sequence shown in SEQ ID NO: 6 was injected into nude mice.

### Experimental group 4

In the present experimental group, zinc finger-like peptide with sequence shown in SEQ ID NO: 6, in which the fourth amino acid serine was phosphorylated, and injected nude mice.

### Control group

In the control group, H₂O (100 µl) was injected into nude mice.

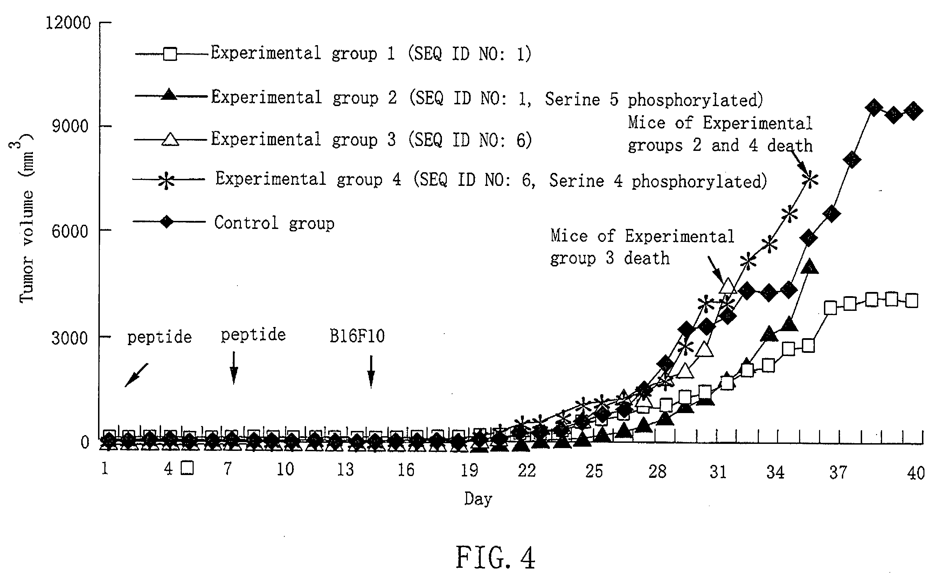

The results of the present example are shown in FIG. 4, which shows curves representing the tumor volumes of mice of the experimental groups 1-4 and the control group of the present example. As shown in FIG. 4, the nude mice of the experimental group 3 died on 31^{st} day, and those of the experimental groups 2 and 4 died on 35^{th} day. These results indicate that the zinc finger-like peptide with sequence shown in SEQ ID NO: 1 (the experimental group 1) can effectively inhibit the growth of tumor cells. In addition, the skin cancer was completely cured after 115 days, and therefore the zinc finger-like peptide with sequence shown in SEQ ID NO: 1 has effect on skin cancer treatment. Furthermore, according to the results of the experimental groups 2-4, the activities of the zinc finger-like peptide on skin cancer treatment were reduced by phosphorylation of serine or deletion of cyctine of zinc finger-like peptide.

### [Example 5]

The cDNA of zinc finger-like peptide was inserted into vector pCR3.1/CMV to form a zinc finger-like peptide expression plasmid, wherein the zinc finger-like peptide was connected to the c-terminal of EGFP.

### Experimental group 1

In the present experimental group, the zinc finger-like peptide expression plasmid containing DNA sequence for expressing the zinc finger-like peptide (SEQ ID NO: 4) (100 µl) was injected into nude mice via tail veins per week for 2 consecutive weeks, to express zinc finger-like peptide. Then, BCC cells (2 million cells / 100 µl medium) were inoculated into nude mice at both the right and left flanks. The tumor volumes (mm³) were observed daily during the whole experiment.

### Experimental group 2

The process of the present experimental group was the same as that of the experimental group 1, except that the DNA sequence shown in SEQ ID NO: 4 was substituted with DNA sequence shown in SEQ ID NO: 7.

### Control group

The process of the present control group was the same as that of the experimental group 1, except that the zinc finger-like peptide expression plasmid was substituted with vector containing EGFP only.

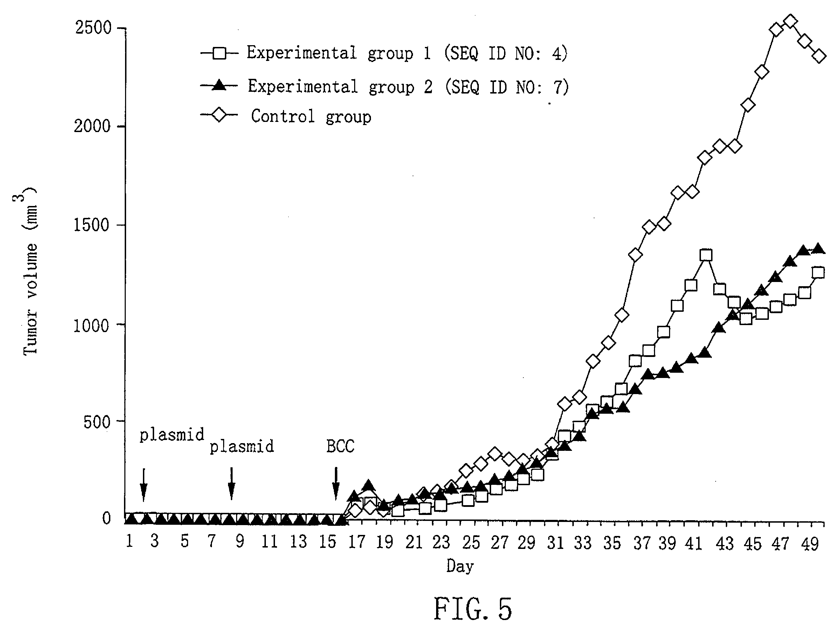

The results of the present example are shown in FIG. 5, which shows curves representing the tumor volumes of mice of the experimental groups 1-2 and the control group of the present example. As shown in FIG. 5, the growth of tumor cells can be inhibited and the volume thereof can be reduced, when the zinc finger-like peptide expression plasmid containing DNA sequence shown in SEQ ID NO: 4 (experimental group 1) was injected into nude mice. Hence, the zinc finger-like peptide expression plasmid exhibits ability to treat skin cancer. Although the zinc finger-like peptide expression plasmid containing DNA sequence shown in SEQ ID NO: 7 (experimental group 2) cannot effectively terminate the growth of tumor cells, it can slightly inhibit the growth of the tumor cells. These results indicate that serine in the zinc finger-like peptide plays an important role on the cancer treatment.

### [Example 6]

### Experimental group

In the present experimental group, zinc finger-like peptide with sequences shown in SEQ ID NO: 8 and 9 was injected into nude mice.

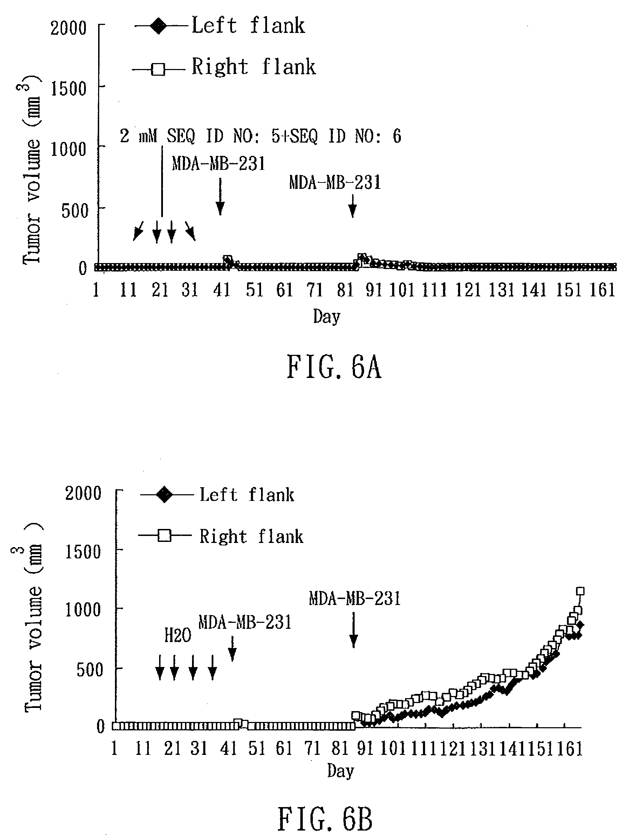

Nude mice received an intravenous injection of peptide (2 mM, 100 µl) via tail veins per week for 4 consecutive weeks, followed by inoculating breast adenocarcinoma MDA-MB-231 (2 million cells / 100 µl medium) at both the right and left flanks on 40^{th} day and 85^{th} day. The tumor volumes (mm³) were observed daily during the whole experiment. The result is shown in FIG. 6A, which is a curve representing the tumor volumes of mice.

### Control group

The process of the present control group was the same as that of the experimental group, except that the zinc finger-like peptide of the experimental group was substituted with H₂O (100 µl) in the present control group. The result is shown in FIG. 6B, which is a curve representing the tumor volumes of mice.

As shown in FIG. 6A, when the nude mice were injected with peptide and followed by inoculating breast tumor cells (experimental group), the growth and proliferation of tumor cells were not observed at both the left and right flanks. However, as shown in FIG. 6B, the tumor volumes of the nude mice in the control group were increased to 1000 times at both the left and right flanks on the 161^{st} day. These results indicate that the zinc finger-like peptide exhibit effects on treating and preventing breast adenocarcinoma.

In addition, there are no side effects on the nude mice injected with the zinc finger-like peptide 1.5 years layer. Hence, the zinc finger-like peptide exhibits low side effect and has ability to prevent the growth of breast tumor cells.

### [Example 7]

### Experimental group

In the present experimental group, zinc finger-like peptide with sequence shown in SEQ ID NO: 1 was injected into nude mice.

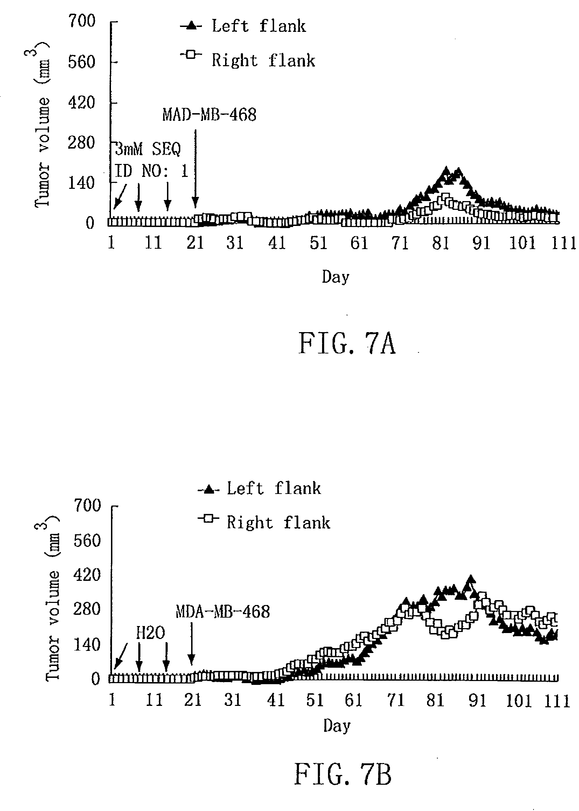

Nude mice were injected with an intravenous injection of peptide (3 mM, 100 µl) via tail veins per week for 3 consecutive weeks, followed by inoculating breast carcinoma MDA-MB-468 (2 million cells / 100 µl medium) at both the right and left flanks. The tumor volumes (mm³) were observed daily during the whole experiment. The result is shown in FIG. 7A, which is a curve representing the tumor volumes of mice.

### Control group

The process of the present control group was the same as that of the experimental group, except that the zinc finger-like peptide of the experimental group as substituted with H₂O (100 µl) in the present control group. The result is shown in FIG. 7B, which is a curve representing the tumor volumes of mice.

As shown in FIG. 7A, when the nude mice were injected with peptide and followed by inoculating breast tumor cells (experimental group), the growth and proliferation of tumor cells were not observed at both the left and right flanks. However, as shown in FIG. 7B, the tumor volumes of the nude mice in the control group were increased to 200-500 times at both the left and right flanks on the 91^{st} day. These results indicate that the zinc finger-like peptide exhibit effects on treating and preventing breast carcinoma.

### [Example 8]

### Experimental group

In the present experimental group, zinc finger-like peptide with sequence shown in SEQ ID NO: 2 was injected subcutaneously into nude mice.

Nude mice received an injection of breast carcinoma MDA-MB-435s (2 million cells / 100 µl PBS) via tail veins at both the right and left flanks on the 1^{st} day, followed by injecting peptide (2 mM, 100 µl) per week for 4 consecutive weeks from the 70^{th} day. The tumor volumes (mm³) were observed daily during the whole experiment.

### Control group

The process of the present control group was the same as that of the experimental group, except that the zinc finger-like peptide of the experimental group was substituted with PBS buffer (100 µl) in the present control group.

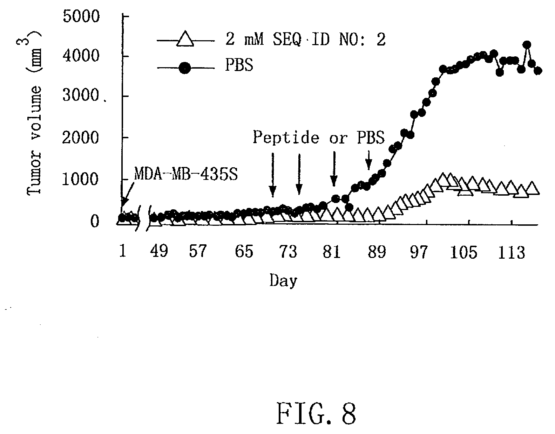

The results of the present example are shown in FIG. 8, which shows curves representing the tumor volumes of mice of the experimental groups 1-2 and the control group of the present example. The tumor volumes in the experimental group were significantly increased from the 89^{th} day, and the maximum tumor volumes were observed on the 100^{th} day (1000 mm³). Then, the tumor volumes were reduced to 50% on the 113^{th} day. However, the tumor volumes in the control group were significantly increased from the 80^{th} day, and increased to 4000 times (4000 mm³) on the 105^{th} day. Although the tumor volumes in the experimental group were increased, the zinc finger-like peptide used therein can still terminate 75% tumor cell growth compared to the control group. Hence, the zinc finger-like peptide can be used to treat breast cancer.

### [Example 9]

### Experimental group

In the present experimental group, zinc finger-like peptide with sequence shown in SEQ ID NO: 1 was injected into nude mice.

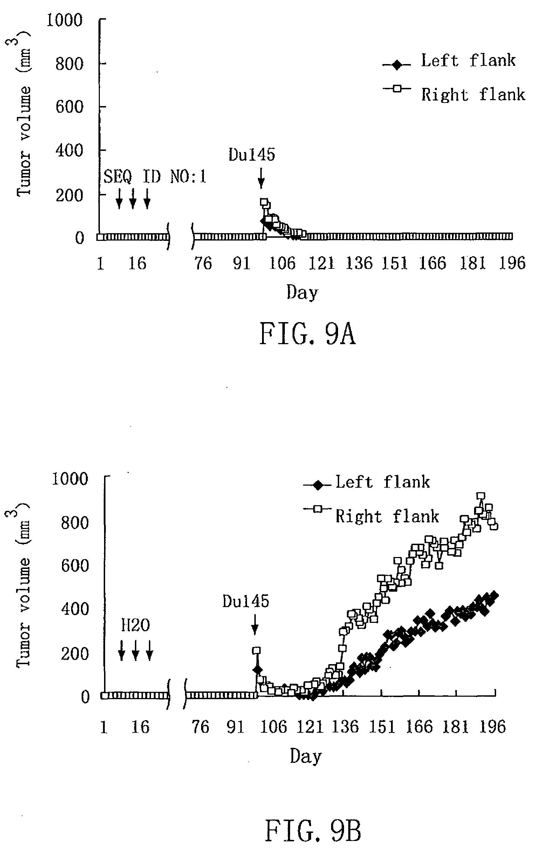

Nude mice were injected with an intravenous injection of peptide (2 mM, 100 µl) via tail veins per week for 3 consecutive weeks, followed by inoculating malignant prostate cancer DU145 cells (2 million cells / 100 µl medium) at both the right and left flanks three months later. The tumor volumes (mm³) were observed daily during the whole experiment. The result is shown in FIG. 9A, which is a curve representing the tumor volumes of mice.

### Control group

The process of the present control group was the same as that of the experimental group, except that the zinc finger-like peptide of the experimental group was substituted with H₂O (100 µl) in the present control group. The result is shown in FIG. 9B, which is a curve representing the tumor volumes of mice.

As shown in FIG. 9A, when the nude mice were injected with peptide and followed by incoluation of prostate tumor cells (experimental group), the growth and proliferation of tumor cells were not observed at both the left and right flanks. In addition, there is a 3-month resting period after peptide treatment prior to challenge with tumor cells, suggesting that zinc finger-like peptide could provide a long-term protection against cancer.

However, as shown in FIG. 9A and FIG. 9B, the implanted tumor cells in the control group without peptide injection proliferated at both the right and left flanks, and the tumor volumes of the control group were much lager than those of the experimental group.

### [Example 10]

### Experimental group

In the present experimental group, zinc finger-like peptide with sequence shown in SEQ ID NO: 1 was injected into nude mice.

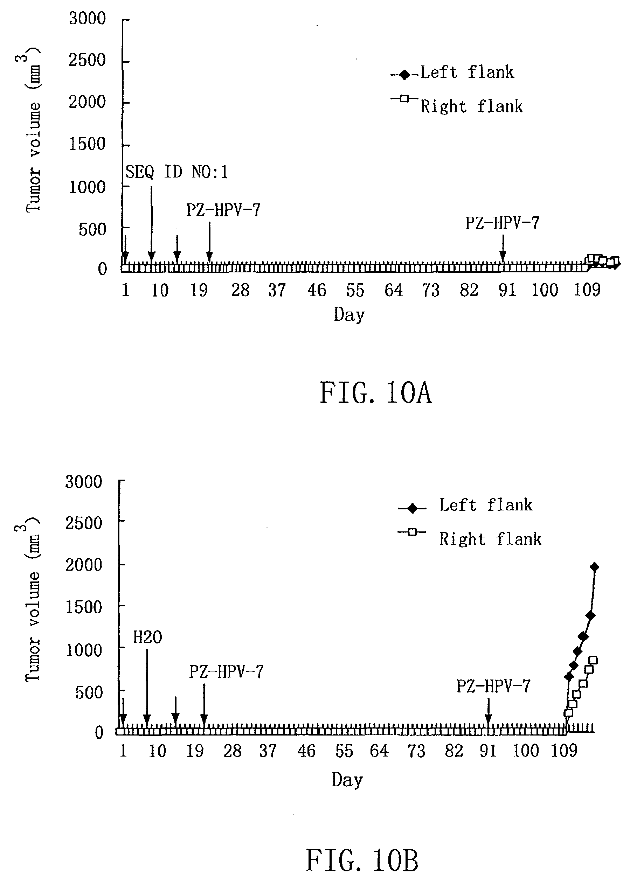

Nude mice were injected with an intravenous injection of peptide (2 mM, 100 µl) via tail veins per week for 3 consecutive weeks, followed by inoculating prostatic epithelial cells PZ-HPV-7 (2 million cells / 100 µl medium) at both the right and left flanks on the 20^{th} day and the 90^{th} day. The tumor volumes (mm³) were observed daily during the whole experiment. The result is shown in FIG. 10A, which is a curve representing the tumor volumes of mice.

### Control group

The process of the present control group was the same as that of the experimental group, except that the zinc finger-like peptide of the experimental group was substituted with H₂O (100 µl) in the present control group. The result is shown in FIG. 10B, which is a curve representing the tumor volumes of mice.

As shown in FIG. 10A, when the nude mice were injected with peptide and followed by inoculating prostate tumor cells (experimental group), the growth and proliferation of tumor cells were not observed at both the left and right flanks. However, as shown in FIG 10B, the implanted tumor cells in the control group without peptide injection were proliferated at both the right and left flanks.

Hence, the zinc finger-like peptide can be used to treat prostate cancer, terminate the growth of tumor cells, and obtain the purpose of cancer prevention.

### [Example 11]

### Control group

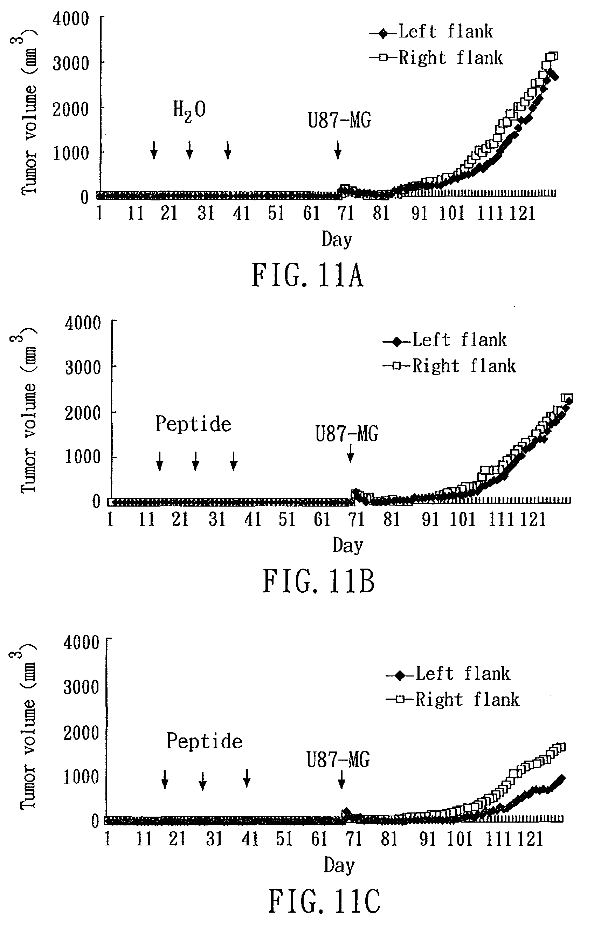

The process of the present control group was the same as that of the experimental group, except that the zinc finger-like peptide of the experimental group was substituted with H₂O (100 µl) in the present control group. The result is shown in FIG. 11A, which is a curve representing the tumor volumes of mice.

### Experimental group 1

In the present experimental group, zinc finger-like peptide with sequence shown in SEQ ID NO: 1 was injected into nude mice.

Nude mice were injected with an intravenous injection of peptide (2 mM, 100 µl) via tail veins per week for 3 consecutive weeks, followed by inoculating malignant glioma U87-MG cells (2 million cells / 100 µl medium) at both the right and left flanks 1 month later. The tumor volumes (mm³) were observed daily during the whole experiment. The result is shown in FIG. 11B, which is a curve representing the tumor volumes of mice.

### Experimental group 2

The process of the present control group was the same as that of the experimental group 1, except that the concentration of the zinc finger-like peptide is 4 mM. The result is shown in FIG 11C, which is a curve representing the tumor volumes of mice.

As shown in FIG. 11A to FIG. 11C, when the nude mice were injected with peptide and followed by inoculating brain tumor cells (experimental groups 1 and 2), the growth and proliferation of tumor cells were slower than that observed in the control group. In addition, the suppression on tumor cell growth was getting significant as the concentration of the zinc finger-like peptide increased.

The aforementioned results indicate that the zinc finger-like peptide or the zinc finger-like peptide expression plasmid can inhibit the growth and the proliferation of tumor cells, and thus has potential for cancer treatment and prevention.

## Claims

1. A zinc finger-like peptide for use in treating cancer, comprising:
at least seven amino acids, wherein the sequence of the at least seven amino acids is represented by SEQ ID NO: 1,
wherein the peptide is to be administered via intravenous injection.

2. The zinc finger-like peptide for use as claimed in claim 1, wherein the sequence of the zinc finger-like peptide is SEQ ID NO: 1.

3. The zinc finger-like peptide for use as claimed in claim 1, wherein the sequence of the zinc finger-like peptide is SEQ ID NO: 2.

4. A pharmaceutical composition for use in treating cancer, comprising:
an effective amount of a zinc finger-like peptide, wherein the zinc finger-like peptide comprises at least seven amino acids, wherein the sequence of the at least seven amino acids is represented by SEQ ID NO: 1; and
a pharmaceutically acceptable carrier,
wherein the composition is to be administered via intravenous injection.

5. The pharmaceutical composition for use as claimed in claim 4, wherein the sequence of the zinc finger-like peptide is SEQ ID NO: 1.

6. The pharmaceutical composition for use as claimed in claim 4, wherein the sequence of the zinc finger-like peptide is SEQ ID NO: 2.

7. The pharmaceutical composition for use as claimed in claim 4, wherein the pharmaceutically acceptable carrier is at least one selected from the group consisting of: activators, excipients, adjuvants, dispersants, wetting agents, and suspensions.

8. The zinc finger-like peptide for use as claimed in claim 1, wherein the cancer is bladder cancer, bone cancer, brain cancer, breast cancer, cervical cancer, colon cancer, endometrial cancer, esophageal cancer, leukemia, liver cancer, lymphoma, kidney cancer, osteosarcoma, ovarian cancer, pancreatic cancer, prostate cancer, skin cancer including basal and squamous cell carcinoma and melanoma, small intestine cancer, stomach cancer, thymus cancer or thyroid cancer.

9. The zinc finger-like peptide for use as claimed in claim 8, wherein the cancer is brain cancer, breast cancer, prostate cancer or skin cancer.

10. The pharmaceutical composition for use as claimed in claim 4, wherein the cancer is bladder cancer, bone cancer, brain cancer, breast cancer, cervical cancer, colon cancer, endometrial cancer, esophageal cancer, leukemia, liver cancer, lymphoma, kidney cancer, osteosarcoma, ovarian cancer, pancreatic cancer, prostate cancer, skin cancer including basal and squamous cell carcinoma and melanoma, small intestine cancer, stomach cancer, thymus cancer or thyroid cancer.

11. The pharmaceutical composition for use as claimed in claim 10, wherein the cancer is brain cancer, breast cancer, prostate cancer or skin cancer.

## Patentansprüche

1. Zinkfingerartiges Peptid für die Verwendung in der Krebsbehandlung, aufweisend:
mindestens sieben Aminosäuren, wobei die Sequenz der mindestens sieben Aminosäuren durch SEQ ID Nr. 1 dargestellt ist,
wobei das Peptid über eine intravenöse Injektion verabreicht werden soll.

2. Zinkfingerartiges Peptid für die Verwendung nach Anspruch 1, wobei die Sequenz des zinkfingerartigen Peptids SEQ ID Nr. 1 ist.

3. Zinkfingerartiges Peptid für die Verwendung nach Anspruch 1, wobei die Sequenz des zinkfingerartigen Peptids SEQ ID Nr. 2 ist.

4. Pharmazeutische Zusammensetzung für die Verwendung in der Krebsbehandlung, aufweisend:
eine wirksame Menge eines zinkfingerartigen Peptids, wobei das zinkfingerartige Peptid mindestens sieben Aminosäuren aufweist, wobei die Sequenz der mindestens sieben Aminosäuren durch SEQ ID Nr. 1 dargestellt ist;
und
einen pharmazeutisch annehmbaren Träger, wobei die Zusammensetzung über eine intravenöse Injektion verabreicht werden soll.

5. Pharmazeutische Zusammensetzung für die Verwendung nach Anspruch 4, wobei die Sequenz des zinkfingerartigen Peptids SEQ ID Nr. 1 ist.

6. Pharmazeutische Zusammensetzung für die Verwendung nach Anspruch 4, wobei die Sequenz des zinkfingerartigen Peptids SEQ ID Nr. 2 ist.

7. Pharmazeutische Zusammensetzung für die Verwendung nach Anspruch 4, wobei der pharmazeutisch annehmbare Träger zumindest einer, ausgewählt aus der Gruppe bestehend aus Aktivatoren, Hilfsstoffen, Adjuvantien, Dispergiermittel, Benetzungsmittel und Suspensionen ist.

8. Zinkfingerartiges Peptid für die Verwendung nach Anspruch 1, wobei der Krebs Blasenkrebs, Knochenkrebs, Gehirntumor, Brustkrebs, Gebärmutterhalskrebs, Darmkrebs, Endometriumkarzinom, Speiseröhrenkrebs, Leukämie, Leberkrebs, Lymphom, Nierenkrebs, Osteosarkom, Eierstockkrebs, Bauchspeicheldrüsenkrebs, Prostatakrebs, Hautkrebs einschließlich Basal- und Plattenepithelkarzinom und Melanom, Dünndarmkrebs, Magenkrebs, Thymuskrebs oder Schilddrüsenkrebs ist.

9. Zinkfingerartiges Peptid für die Verwendung nach Anspruch 8, wobei der Krebs Gehirntumor, Brustkrebs, Prostatakrebs oder Hautkrebs ist.

10. Pharmazeutische Zusammensetzung für die Verwendung nach Anspruch 4, wobei der Krebs Blasenkrebs, Knochenkrebs, Gehirntumor, Brustkrebs, Gebärmutterhalskrebs, Darmkrebs, Endometriumkarzinom, Speiseröhrenkrebs, Leukämie, Leberkrebs, Lymphom, Nierenkrebs, Osteosarkom, Eierstockkrebs, Bauchspeicheldrüsenkrebs, Prostatakrebs, Hautkrebs einschließlich Basal- und Plattenepithelkarzinom und Melanom, Dünndarmkrebs, Magenkrebs, Thymuskrebs oder Schilddrüsenkrebs ist.

11. Pharmazeutische Zusammensetzung für die Verwendung nach Anspruch 10, wobei der Krebs Gehirntumor, Brustkrebs, Prostatakrebs oder Hautkrebs ist.

## Revendications

1. Peptide de type en doigt de zinc pour une utilisation de traitement du cancer, comprenant :
au moins sept acides aminés, dans lequel la séquence des au moins sept acides aminés est représentée par SEQ ID NO: 1,
dans lequel le peptide est à administrer par injection intraveineuse.

2. Peptide de type en doigt de zinc pour une utilisation selon la revendication 1, dans lequel la séquence du peptide de type en doigt de zinc est SEQ ID NO: 1.

3. Peptide de type en doigt de zinc pour une utilisation selon la revendication 1, dans lequel la séquence du peptide de type en doigt de zinc est SEQ ID NO: 2.

4. Composition pharmaceutique pour une utilisation de traitement du cancer, comprenant :
une quantité efficace d'un peptide de type en doigt de zinc, dans laquelle le peptide de type en doigt de zinc comprend au moins sept acides aminés, dans laquelle la séquence des au moins sept acides aminés est représentée par SEQ ID NO: 1 ; et
un vecteur pharmaceutiquement acceptable,
dans laquelle la composition est à administrer par injection intraveineuse.

5. Composition pharmaceutique pour une utilisation selon la revendication 4, dans laquelle la séquence du peptide de type en doigt de zinc est SEQ ID NO: 1.

6. Composition pharmaceutique pour une utilisation selon la revendication 4, dans laquelle la séquence du peptide de type en doigt de zinc est SEQ ID NO: 2.

7. Composition pharmaceutique pour une utilisation selon la revendication 4, dans laquelle le vecteur pharmaceutiquement acceptable est au moins un sélectionné dans le groupe constitué : d'activateurs, d'excipients, d'adjuvants, de dispersants, d'agents mouillants et de suspensions.

8. Peptide de type en doigt de zinc pour une utilisation selon la revendication 1, dans lequel le cancer est un cancer de la vessie, un cancer des os, un cancer du cerveau, un cancer du sein, un cancer du col de l'utérus, un cancer du côlon, un cancer de l'endomètre, un cancer de l'oesophage, une leucémie, un cancer du foie, un lymphome, un cancer du rein, un ostéosarcome, un cancer de l'ovaire, un cancer du pancréas, un cancer de la prostate, un cancer de la peau incluant le mélanome et le carcinome basocellulaire et spinocellulaire, un cancer de l'intestin grêle, un cancer de l'estomac, un cancer du thymus ou un cancer de la thyroïde.

9. Peptide de type en doigt de zinc pour une utilisation selon la revendication 8, dans lequel le cancer est un cancer du cerveau, un cancer du sein, un cancer de la prostate ou un cancer de la peau.

10. Composition pharmaceutique pour une utilisation selon la revendication 4, dans laquelle le cancer est un cancer de la vessie, un cancer des os, un cancer du cerveau, un cancer du sein, un cancer du col de l'utérus, un cancer du côlon, un cancer de l'endomètre, un cancer de l'oesophage, une leucémie, un cancer du foie, un lymphome, un cancer du rein, un ostéosarcome, un cancer de l'ovaire, un cancer du pancréas, un cancer de la prostate, un cancer de la peau incluant le mélanome et le carcinome basocellulaire et spinocellulaire, un cancer de l'intestin grêle, un cancer de l'estomac, un cancer du thymus ou un cancer de la thyroïde.

11. Composition pharmaceutique pour une utilisation selon la revendication 10, dans laquelle le cancer est un cancer du cerveau, un cancer du sein, un cancer de la prostate ou un cancer de la peau.
